# EUROPEAN PATENT APPLICATION

(11) **EP 1 258 258 A1**
(43) Date of publication of application: **20.11.2002**
(21) Application number: 02010498.0
(22) Date of filing: 08.05.2002
(51) Int. Cl.: A61L 31/16, A61L 31/14

(54) **Implantable device comprising a stent and angiotensin II receptor antagonists**

(30) Priority: 08.05.2001 JP 2001136966
(71) Applicant: TERUMO KABUSHIKI KAISHA, Tokyo (JP)
(72) Inventor: Sugimoto, Ryota, Ashigarakami-gun, Kanagawa (JP)
(74) Representative: Casalonga, Axel

(57) **Abstract**

There are provided an implantable medical material composed of an angiotensin II receptor antagonist and a biocompatible material or a biodegradable material, which can be applied to an injury lesion of the blood vessel directly and locally, and can reliably suppress the proliferation of smooth muscle cells and prevent restenosis of the blood vessel and an implantable medical device composed of the medical material and a holder or a stent.

## Description

### BACKGROUND OF THE INVENTION

### 1. Field of the Invention

The present invention relates to an implantable medical material used for healing a stenosis portion formed in a living body such as the blood vessel, the bile duct, the trachea, the esophagus, and the urethra, and implantable medical device using the implantable medical material.

### 2. Description of the Related Art

An example of a holder for holding an implantable medical material includes a stent. The stent is used for maintaining a stenosis portion formed in the blood vessel or in other lumens in a living body in an expanded state, and is used, for example, for healing a stenosis portion after percutaneous transluminal coronary angioplasty (PTCA). The stent is classified into a self-expandable stent and a balloon expandable stent, depending on the function and implanting method.

In the case of the balloon expandable stent, a stent itself does not have an expanding function. After the balloon expandable stent is inserted into a target lesion, the stent allows a balloon disposed therein to expand to a substantially normal lumen diameter of the target lesion, and the stent is enlarged (plastic-deformation) by an expansion force of the balloon, whereby the stent is fixed on an inner surface of the target lesion in a close contact state. However, after the stent is implanted, the inside of the stent is narrowed again in a ratio of 20 to 40% (restenosis phenomenon).

Various theories have been proposed regarding the cause of restenosis. At present, restenosis is considered to occur as follows: after the stent is implanted, smooth muscle cells of a blood vessel media migrate/proliferate to the intimal side to cause the intimal hyperplasia, whereby restenosis occurs.

Various attempts have been made to prevent restenosis by mounting a drug capable of suppressing migration/proliferation of smooth muscle cells on a stent.

For example, there are a method of suppressing restenosis by allowing a stent to contain taxol described in JP 9-503488 A, a method of suppressing restenosis by allowing a stent to contain mitomycin C, adriamycin, genistein, thyrphostin, or the like described in JP 9-56807 A, a method of suppressing restenosis by allowing a stent to contain cytochalasin described in JP 11-500635 A, and the like.

On the other hand, an angiotensin II receptor antagonist is a receptor antagonist of angiotensin II that is an effective hormone of a renin-angiotensin (RA) type, which is used as a hypotensor. Angiotensin II is the strongest vasopressor substance for maintaining circulation conation in a center system/periphery system via a specific receptor. As the action of an angiotensin-converting enzyme (ACE) inhibitor on a cardiovascular morbidity is being clarified, in addition to the presence of a tissue RA and cell proliferation action of angiotensin II, inhibiting the action of angiotensin II is becoming a first choice for curing a hypertensive/cardiovascular morbidity.

It has been made clear that an angiotensin II receptor has four sub-types. In particular, the actions of an AT1 receptor and an AT2 receptor have been substantially made clear. It is found that the AT1 receptor is present mainly in the blood vessel, the liver, the adrenal cortex, and the kidney, and participates in all the actions of traditional angiotensin II conventionally recognized, such as vasoconstriction, aldosterone secretion, hypertrophy of the cardiac muscle, cell proliferation, and catecholamine isolation (Inagami, T. and Kitami, Y.: Hypertens. Res. 17: 87-97, 1994).

Particularly, in the cardiovascular system, the AT1 receptor participates in the hypertrophy of cardiac muscle cells/blood vessel smooth muscle cells, acceleration of growth of fibroblast, stimulus of production of fibronectin/collagen, and the like. When a cardiovascular system is reconstructed, expression of AT1 is further increased. An angiotensin II receptor antagonist under development is a drug that selectively antagonizes the AT1 receptor. Due to the use of the angiotensin II receptor antagonist, the original antihypertensive effect can be exhibited. By antagonizing the AT1 receptor, proliferation of blood vessel smooth muscle cells is suppressed. Furthermore, the suppression of intimal hyperplasia can be expected.

Furthermore, it is found that the angiotensin II receptor antagonist also acts on the AT2 receptor. The action of the AT2 receptor is not completely clarified; however, it is considered that the AT2 receptor has a function of antagonizing the AT1 receptor, such as suppressing the cell proliferation, accelerating apotosis, vasodilation action, and the like (Nakajima, M. et al., Proc. Natl. Acad. Sci. USA. 82: 10663-10667, 1995).

Recently, it has been reported that a risk of restenosis can be reduced by implanting a stent in a stenosis portion of a subject and allowing the subject to take valsartan that is one of the angiotensin II receptor antagonists.

However, according to the above-mentioned report, a risk of restenosis was reduced only by orally administering an angiotensin II receptor antagonist to the subject. Therefore, means for directly and locally applying the angiotensin II receptor antagonist to an injury lesion such as the blood vessel has not yet been known.

### SUMMARY OF THE INVENTION

Therefore, with the foregoing in mind, it is an object of the present invention to provide an implantable medical material or an implantable medical device using the implantable medical material capable of being applied to an injury lesion such as the blood vessel directly and locally, reliably suppressing the proliferation of smooth muscle cells, and preventing restenosis of the blood vessel and the like.

The above object will be achieved according to the following (1) to (7) of the present invention.
(1) There is provided an implantable medical material characterized by comprising an angiotensin II receptor antagonist and a biocompatible material or a biodegradable material.
(2) There is provided an implantable medical material according to (1), characterized in that the angiotensin II receptor antagonist is contained in a biocompatible material or a biodegradable material.
(3) There is provided an implantable medical material according to claim (1) or (2), characterized in that the angiotensin II receptor antagonist is one selected from the group consisting of losartan potassium, candesartan cilexetil, valsartan, telmisartan, zolasartan, irbesartan, eprosartan, olmesartan, and embusartan.
(4) There is provided an implantable medical material according to any one of (1) to (3), characterized in that the biocompatible material is silicone.
(5) There is provided an implantable medical material according to any one of (1) to (3), characterized in that the biodegradable material is one selected from the group consisting of polylactic acid, polyglycolic acid, a polylactic acid-polyglycolic acid copolymer, and polyhydroxybutylic acid.
(6) There is provided an implantable medical device, characterized by comprising the implantable medical material of any one of claims 1 to 5, and a holder for holding the implantable medical material.
(7) There is provided an implantable medical device according to (6), characterized in that the holder is a stent.

These and other advantages of the present invention will become apparent to those skilled in the art upon reading and understanding the following detailed description with reference to the accompanying figures.

### BRIEF DESCRIPTION OF THE DRAWINGS

In the accompanying drawings:
FIG. 1 is a cross-sectional view of an implantable medical device according to the present invention; and
FIG. 2 is a cross-sectional view of another implantable medical device according to the present invention.

### DESCRIPTION OF THE PREFERRED EMBODIMENTS

Hereinafter, an implantable medical material of the present invention will be described.

The implantable medical material of the present invention is composed of an angiotensin II receptor antagonist and a biocompatible material or a biodegradable material.

There is no particular limit to the angiotensin II receptor antagonist of the present invention as long as it is a receptor antagonist of angiotensin II. Examples of the angiotensin II receptor antagonist include losartan potassium, candesartan cilexetil, valsartan, telmisartan, zolasartan, irbesartan, eprosartan, olmesartan and embusartan. Among these, candesartan cilexetil and valsartan are preferable.

There is no particular limit to the biocompatible material of the present invention, as long as it is unlikely to allow the platelet to adhere thereto, does not exhibit stimulus to a tissue, and is capable of eluting the angiotensin II receptor antagonist. Examples of the biocompatible material include various synthetic polymers such as a blend of polyether polyurethane and dimethyl silicone or a block copolymer, polyurethane (e.g., segmented polyurethane), polyacrylamide, polyethylene oxide, polycarbonate (e.g., polyethylene carbonate, polypropylene carbonate), silicone and polymethoxyethyl acrylate. Among these, silicone is preferable.

There is no particular limit to the biodegradable material of the present invention, as long as it is decomposed in a living body enzymatically or non-enzymatically, its decomposed product does not exhibit toxicity, and it is capable of releasing the angiotensin II receptor antagonist. Examples of the biodegradable material include polylactic acid, polyglycolic acid, polylactic acid-polyglycolic acid copolymer, polyhydroxybutylic acid, polymalic acid, polyα-amino acid, collagen, laminin, heparan sulfate, fibronectin, hydronectin, chondroitin sulfate, hyaluronic acid, chitin, chitosan and the like. Among these, polylactic acid and polyglycolic acid are preferable.

The implantable medical material of the present invention is composed of an angiotensin II receptor antagonist and a biocompatible material or a biodegradable material. There is no particular limit to the form of composition thereof. The angiotensin II receptor antagonist may be contained (mixed) in the biocompatible material or the biodegradable material. Alternatively, the angiotensin II receptor antagonist and the biocompatible material or the biodegradable material may be present as separate layers without being mixed.

In the case where the angiotensin II receptor antagonist is contained in the biocompatible material or the biodegradable material, there is no particular limit to the form thereof. The angiotensin II receptor antagonist may be present in the biocompatible material or the biodegradable material uniformly or non-uniformly, or may be present locally. However, in order to enhance the function of suppressing the proliferation of smooth muscle cells that the angiotensin II receptor antagonist has, it is preferable that the angiotensin II receptor antagonist is uniformly dispersed in a portion of the biocompatible material or the biodegradable material contacting an injury lesion of the blood vessel and the like. The content of the angiotensin II receptor antagonist is appropriately determined without any particular limit.

There is no particular limit to a method of producing an implantable medical material of the present invention. For example, in the case where polylactic acid is used as a biodegradable material, and valsartan is used as the angiotensin II receptor antagonist, respectively, a solution in which polylactic acid is dissolved in dichloromethane is mixed with a solution in which valsartan is dissolved in ethanol. Thereafter, the mixture is dropped onto water that has been stirred at a high speed to obtain a suspension of fine particles of polylactic acid in which valsartan is contained in a uniformly dispersed state. The fine particles are extracted from the suspension, whereby an implantable medical material of the present invention can be obtained.

There is no particular limit to a method of using an implantable medical material of the present invention, as long as it is a method of directly applying a material to an injury lesion of the blood vessel and the like. For example, there are the method of directly applying a catheter or a balloon provided with the implantable medical material of the present invention on its surface to an injury lesion after a stenosis lesion is expanded, and the method of allowing a holder for holding the implantable medical material of the present invention to implant in an injury lesion after a stenosis lesion is expanded, and the like.

The method of directly applying the implantable medical material of the present invention by using a catheter or a balloon provided with the material is preferable for healing a complicated injury lesion in which it is difficult to allow the holder such as a stent to implant. A method of allowing a holder for holding the implantable medical material of the present invention to implant is preferable for blood vessel remodeling in which the inner lumen of the blood vessel is narrowed.

The implantable medical material of the present invention is composed of the angiotensin II receptor antagonist and the biocompatible material or the biodegradable material. Therefore, it can be directly applied to an injury lesion of the blood vessel and the like.

In the case where the angiotensin II receptor antagonist is contained in the biocompatible material, the angiotensin II receptor antagonist is eluted to the outer surface of the biocompatible material, whereby the angiotensin II receptor antagonist is directly released to an injury lesion of the blood vessel and the like. Therefore, the proliferation of smooth muscle cells can be suppressed, and restenosis can be prevented.

Furthermore, by dispersing the angiotensin II receptor antagonist to the inside of the biocompatible material, a substained-release property of the angiotensin II receptor antagonist can be provided. Because of this, the angiotensin II receptor antagonist can be eluted little by little for a long period of time in a continuous manner.

In the case where the angiotensin II receptor antagonist is contained in the biodegradable material, the biodegradable material is decomposed, whereby the angiotensin II receptor antagonist is directly released to an injury lesion of the blood vessel and the like. Therefore, the proliferation of smooth muscle cells can be suppressed, and restenosis can be prevented.

Furthermore, by dispersing the angiotensin II receptor antagonist to the inside of the biodegradable material, a substained-release property of the angiotensin II receptor antagonist can be provided. Because of this, the angiotensin II receptor antagonist can be released little by little for a long period of time in a continuous manner.

In the case where the angiotensin II receptor antagonist and the biocompatible material or the biodegradable material have a two-layer composition or a multi-layer composition, instead of being mixed (for example, in the case where the angiotensin II receptor antagonist is present in a layer shape in the lower layer, and the biocompatible material or the biodegradable material is present in a layer shape in the upper layer), the angiotensin II receptor antagonist is eluted after passing through the biocompatible material or the biodegradable material in the upper layer. Therefore, the angiotensin II receptor antagonist can be released little by little for a long period of time in a continuous manner.

The implantable medical material of the present invention can be mixed with a drug capable of suppressing the hyperplasia and restenosis in addition to the angiotensin II receptor antagonist. Examples of the drug mentioned above include an anticancer agent, an immunosuppressive agent, an HMG - CoA reductase inhibitor, an ACE inhibitor, a Ca-antagonist, an anti-allergic drug, an antioxidant, and the like. Due to the synergistic effect of the above with the angiotensin II receptor antagonist, it becomes possible to suppress the hyperplasia.

Hereinafter, an implantable medical device of the present invention will be described.

The implantable medical device of the present invention includes the above-mentioned implantable medical material of the present invention and a holder for holding the implantable medical material.

There is no particular limit to the material, shape, size, and the like of the holder of the present invention, as long as it can hold an implantable medical material, and is allowed to implant safely in a lumen such as the blood vessel.

Examples of the material for the holder include various inorganic compounds, various organic compounds, composite materials thereof, and the like.

Examples of the inorganic material include stainless steel, an Ni-Ti alloy, various kinds of metals such as tantalum, ceramics, and the like. Examples of the organic compound include polytetrafluoroethylene, polyethylene, polypropylene, polyethylene terephthalate, and the like.

There is no particular limit to the shape of the holder, as long as it has strength sufficient for implant safely in a lumen such as the blood vessel. For example, an arbitrary shape such as a cylinder made of a mesh body composed of wires made of an inorganic compound, or organic compound fibers, and a cylinder made of an inorganic compound or an organic compound having pores are preferably used.

Furthermore, the implantable medical device of the present invention can be formed as a holder such as a stent, a catheter, a balloon, a blood vessel prosthetic material, an artificial blood vessel, and the like. Among them, it is preferable that the holder is a stent.

The stent can be formed, for example, in a coil shape, a mesh cylinder shape, or the like. The stent may also be either a balloon-expandable type or a self-expandable type. The stent may be either stiff or bendable. Furthermore, the size of the stent may be selected depending on an application portion. Generally, the stent has an inner diameter of preferably 1.0 to 3.0 mm, and a length of 5 to 50 mm.

There is no particular limit to a holding form of the implantable medical material. In terms of safety in carrying to an injury lesion of the blood vessel and the like and safety under the condition implanted in the injury lesion and the like, it is preferable that the implantable medical material is integrated with the holder.

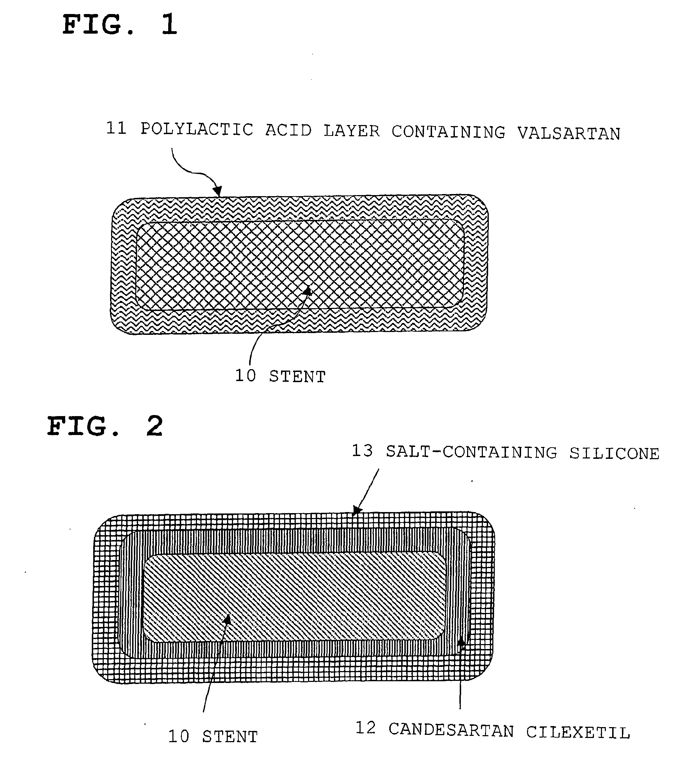

There is no particular limit to a method of integrating the implantable medical material with the holder. For example, as shown in FIG. 1, in the case where polylactic acid is used as a biodegradable material constituting the implantable medical material and valsartan is used as the angiotensin II receptor antagonist, respectively, there is given a method in which, a solution in which polylactic acid is dissolved in dichloroethane is mixed with a solution in which valsartan is dissolved in ethanol, and then, the mixture is sprayed onto the surface of a holder such as a stent 10, thereby integrating the stent 10 with a polylactic acid layer 11 containing valsartan, and the like.

Furthermore, for example, as shown in FIG. 2, in the case where silicone is used as the biocompatible material constituting the implantable medical material, and candesartan cilexetil is used as the angiotensin II receptor antagonist, respectively, there is given a method in which, a solution in which candesartan cilexetil is dissolved in dimethylsulfoxide is sprayed onto the surface of the holder such as a stent 10, and thereafter, a solution in which salt-containing silicone is dissolved in hexane is sprayed onto candesartan cilexetil, thereby integrating the stent 10 with candesartan cilexetil 12 and salt-containing silicone 13 in a two-layer state, and the like.

The implantable medical device of the present invention obtained by these methods can implant directly in an injury lesion of the blood vessel and the like. There is no particular limit to the implanting method, and an example of the implanting method includes a method using a balloon catheter.

In the case where the angiotensin II receptor antagonist is contained in the biocompatible material, the angiotensin II receptor antagonist is eluted to the outer surface of the biocompatible material, whereby the angiotensin II receptor antagonist is directly released to an injury lesion of the blood vessel and the like. Therefore, the proliferation of smooth muscle cells can be suppressed, and restenosis can be prevented.

Furthermore, in the case where the angiotensin II receptor antagonist is contained in the biodegradable material, the biodegradable material is decomposed, whereby the angiotensin II receptor antagonist is directly released to an injury lesion of the blood vessel and the like. Therefore, the proliferation of smooth muscle cells can be suppressed, and restenosis can be prevented.

In the case where the angiotensin II receptor antagonist and the biocompatible material or the biodegradable material are not mixed, but are formed in a two-layer composition or a multi-layer composition (for example, in the case where the angiotensin II receptor antagonist is present in the lower layer, and the biocompatible material or the biodegradable material is present in the upper layer), the angiotensin II receptor antagonist is eluted after passing through the biocompatible material or the biodegradable material in the upper layer. Therefore, the angiotensin II receptor antagonist can be released little by little for a long period of time in a continuous manner.

Furthermore, the implantable medical device of the present invention is provided with a holder for holding an implantable medical material, so that it can apply an implantable medical material to an injury lesion of the blood vessel and the like for a long period of time in a stable manner, and the angiotensin II receptor antagonist can be released to an injury lesion for a long period of time.

### Examples

Hereinafter, the present invention will be described in more detail by way of illustrative examples. Further, it should be noted that the present invention is not limited to the following examples.

### Example 1

A solution in which 40 mg of valsartan is dissolved in 1 ml of ethanol was mixed with a solution in which 40 mg of polylactic acid is dissolved in 4 ml of dichloroethane. The resultant mixed solution was sprayed onto a mesh stent in a cylindrical shape with an inner diameter of 2 mm and a length of 1 cm produced by weaving a stainless wire with a diameter of 50 µm, so as to apply polylactic acid containing valsartan to the mesh stent. As a result, an implantable medical device of the present invention including an implantable medical material of the present invention and a holder for holding the same was produced.

### Comparative Example 1

First, 1 ml of ethanol was mixed with a solution in which 40 mg of polylactic acid is dissolved in 4 ml of dichloroethane. The resultant mixed solution was sprayed onto a mesh stent in a cylindrical shape with an inner diameter of 2 mm and a length of 1 cm produced by weaving a stainless wire with a diameter of 50 µm, thereby applying polylactic acid to the stent.

### Example 2

A solution in which 40 mg of candesartan cilexetil is dissolved in 1 ml of dimethylsulfoxide was mixed with a solution in which 40 mg of polylactic acid is dissolved in 4 ml of dichloroethane. Then, the resultant mixed solution was sprayed onto a stent produced by providing pores on a stainless tubular body with an inner diameter of 2 mm and a length of 1.5 cm, thereby applying polylactic acid containing candesartan cilexetil to the stent. As a result, an implantable medical device of the present invention including an implantable medical material of the present invention and a holder for holding the same was produced.

### Comparative Example 2

A solution in which 40 mg of polylactic acid is dissolved in 4 ml of dichloroethane was obtained. Then, the resultant solution was sprayed onto a stent produced by providing pores on a stainless tubular body with an inner diameter of 2 mm and a length of 1.5 cm, thereby applying polylactic acid to the stent.

### Comparative Example 3

A solution in which 40 mg of rapamycin is dissolved in 1 ml of dichloromethane was mixed with a solution in which 40 mg of polylactic acid is dissolved in 4 ml of dichloroethane. Then, the resultant mixed solution was sprayed onto a stent produced by providing pores on a stainless tubular body with an inner diameter of 2 mm and a length of 1.5 cm, thereby applying polylactic acid containing rapamycin to the stent. As a result, an implantable medical device including the implantable medical material and a holder for holding the same was produced.

### Evaluation Test 1

### Test on the therapeutic effect using a vascular injury model by balloon abrasion of rabbit iliac artery (Example 1/Comparative Example 1)

Kbs: three JW rabbits were anesthetized by intramuscular administration of ketamine (30 mg/kg) and xylazine (3 mg/kg). Right and left femoral arteries were denudated from tissues, and about 150 U/kg of heparin was introduced through auricular veins. Thereafter, a PTCA balloon previously filled with a guide wire was inserted into the blood vessel under fluoroscopy, and transported to the proximal portion of the iliac artery. Under the condition that the balloon was expanded to a specified pressure, the balloon was pulled to the distal portion of the iliac artery, whereby the blood vessel was abraded. This balloon abrasion was repeated three times. The femoral artery was ligated after the removal of the balloon, and three layers were sutured. Abrasion was conducted with respect to both blood vessels of right and left iliac arteries per rabbit. For two weeks after the operation, the rabbits were fed with 1% cholesterol additive. Because of this, the intimal hyperplasia was introduced into the rabbit iliac artery.

Two weeks later, the rabbits were anesthetized by intramuscular administration of ketamine (30 mg/kg) and xylazine (3 mg/kg). The right carotid artery was denudated from tissues. About 150 U/kg of heparin was introduced through auricular veins. Thereafter, a sheath introducer was introduced by a predetermined method. A PTCA balloon previously filled with a guide wire was inserted into the blood vessel, and transported to the distal portion of the iliac artery. Under the condition that the balloon was expanded to a specified pressure, the balloon was pulled to the proximal portion of the iliac artery, whereby the blood vessel was abraded. This balloon abrasion was repeated three times. Then, a stent containing valsartan produced in Example 1 mentioned above was introduced to the right iliac artery, and the stent was allowed to implant in an expanded manner at a specified pressure. Subsequently, the stent produced in Comparative Example 1 mentioned above was introduced to the left iliac artery as a control, whereby the stent was allowed to implant in an expanded manner at a specified pressure. For four weeks after the operation, the rabbits were fed with 0.5% cholesterol additive.

Four weeks later, the rabbits were anesthetized by intramuscular administration of ketamine (30 mg/kg) and xylazine (3 mg/kg). The left carotid artery was denudated from tissues. About 150 U/kg of heparin was introduced through auricular veins. Thereafter, a sheath introducer was introduced by a predetermined method. A diagnostic catheter was inserted into the blood vessel. The right and left iliac arteries were visualized, and abdominal vena cava was exposed by laparotomy. Perfusion was started with 2 U/ml of heparinized saline through a carotid artery sheath line. Simultaneously, abdominal vena cava was cut, whereby the rabbits were exsanguinated and dyed. After general perfusion with heparinized saline, general perfusion was conducted with 10% neutral buffered formalin solution and the target blood vessel was fixed. The fixed sample was embedded in a resin in accordance with a normal method to prepare a pathological section, and the section was subjected to hematoxylin and eosin stain. The resultant pathological section was observed by an optical microscope, whereby the thickness of an intimal hyperplasia was measured.

As a result, the hyperplasia thickness of the left iliac artery in which the stent produced in Comparative Example 1 was allowed to implant was 593 ± 82 µm (n = 3), whereas the hyperplasia thickness of the right iliac artery in which the stent containing valsartan produced in Example 1 was allowed to implant was 207 ± 45 µm (n = 3). From this result, it was confirmed that the intimal hyperplasia of the blood vessel can be suppressed significantly (p < 0.05) by allowing polylactic acid to contain valsartan.

### Evaluation Test 2

### Comparative test on the therapeutic effect with respect to another drug, using a vascular injury model by balloon abrasion of rabbit iliac artery (Example 2/Comparative Examples 2, 3)

Kbs: three JW rabbits were anesthetized by intramuscular administration of ketamine (30 mg/kg) and xylazine (3 mg/kg). Right carotid artery was denudated from tissues, and about 150 U/kg of heparin was introduced through auricular veins. Thereafter, a sheath introducer was introduced by a predetermined method. A PTCA balloon previously filled with a guide wire was inserted into the blood vessel, and transported to the distal portion of the iliac artery. Under the condition that the balloon was expanded to a specified pressure, the balloon was pulled to the proximal portion of the iliac artery, whereby the blood vessel was abraded. This balloon abrasion was repeated three times. Subsequently, the stent containing candesartan cilexetil produced in Example 2 mentioned above, the stent produced in Comparative Example 2, or the stent containing rapamycin produced in Comparative Example 3 was introduced into the right iliac artery, and was allowed to implant in an expanded manner at a specified pressure. The carotid artery was ligated after the removal of the balloon, and three layers were sutured.

Four weeks later, the rabbits were anesthetized by intramuscular administration of ketamine (30 mg/kg) and xylazine (3 mg/kg). The left carotid artery was denudated from tissues. About 150 U/kg of heparin was introduced through auricular veins. Thereafter, a sheath introducer was introduced by a predetermined method. A diagnostic catheter was inserted into the blood vessel. The right and left iliac arteries were visualized, and abdominal vena cava was exposed by laparotomy. Perfusion was started with 2 U/ml of heparinized saline through a carotid artery sheath line. Simultaneously, abdominal vena cava was cut, whereby the rabbits were exsanguinated and dyed. After general perfusion with heparinized saline, general perfusion was conducted with 10% neutral buffered formalin solution and the target blood vessel was fixed. The fixed sample was embedded in a resin in accordance with a normal method to prepare a pathological section, and the section was subjected to hematoxylin and eosin stain. The resultant pathological section was observed by an optical microscope, whereby the thickness of an intimal hyperplasia was measured.

As a result, the hyperplasia thickness of the left iliac artery in which the stent produced in Comparative Example 2 was allowed to implant was 218 ± 26 µm (n = 3), and the hyperplasia thickness of the right iliac artery in which the stent containing rapamycin produced in Comparative Example 3 was allowed to implant was 212 ± 22 µm (n = 3), whereas the hyperplasia thickness of the right iliac artery in which the stent containing candesartan cilexetil produced in Example 2 was allowed to implant was 139 ± 19 µm (n = 3).

From this result, it was confirmed that a significant difference is not found in the hyperplasia thickness of the blood vessel in comparison of the control (Comparative Example 2) with stent containing rapamycin (Comparative Example 3) (218 ± 26 vs. 212 ± 22 µm), whereas the intimal hyperplasia of the blood vessel can be suppressed significantly (p < 0.05) by allowing polylactic acid to contain candesartan cilexetil (218 ± 26 vs. 139 ± 19 µm).

As described above, the implantable medical material of the present invention is characterized by being composed of an angiotensin II receptor antagonist and a biocompatible material or a biodegradable material. Therefore, the implantable medical material is applicable to an injury lesion of the blood vessel and the like directly and locally. Furthermore, the proliferation of smooth muscle cells can be suppressed reliably, and restenosis can be prevented in the blood vessel and the like.

Furthermore, in the case where it is characterized in that the angiotensin II receptor antagonist is either one of losartan potassium, candesartan cilexetil, valsartan, telmisartan, zolasartan, irbesartan, eprosartan, olmesartan, and embusartan, the functional effect of suppressing the proliferation of smooth muscle cells can be exhibited more remarkably.

Furthermore, in the case where it is characterized in that the biodegradable material is either one of polylactic acid, polyglycolic acid, polylactic acid-polyglycolic acid copolymer, and polyhydroxybutylic acid, the biodegradable material exhibits excellent safety to a living body, and can release the angiotensin II receptor antagonist for a long period of time.

Furthermore, in the case where it is characterized in that the present invention includes the implantable medical material and a holder for holding the implantable medical material, the implantable medical material is allowed to implant in an injury lesion of the blood vessel and the like stably for a long period of time. Therefore, the angiotensin II receptor antagonist can be released to an injury lesion reliably.

Various other modifications will be apparent to and can be readily made by those skilled in the art without departing from the scope and spirit of this invention. Accordingly, it is not intended that the scope of the claims appended hereto be limited to the description as set forth herein, but rather that the claims be broadly construed.

## Claims

1. An implantable medical material comprising an angiotensin II receptor antagonist and a biocompatible material or a biodegradable material.

2. An implantable medical material according to claim 1, wherein the angiotensin II receptor antagonist is contained in a biocompatible material or a biodegradable material.

3. An implantable medical material according to claim 1 or 2, wherein the angiotensin II receptor antagonist is one selected from the group consisting of losartan potassium, candesartan cilexetil, valsartan, telmisartan, zolasartan, irbesartan, eprosartan, olmesartan, and embusartan.

4. An implantable medical material according to any one of claims 1 to 3, wherein the biocompatible material is silicone.

5. An implantable medical material according to any one of claims 1 to 3, wherein the biodegradable material is one selected from the group consisting of polylactic acid, polyglycolic acid, a polylactic acid-polyglycolic acid copolymer, and polyhydroxybutylic acid.

6. An implantable medical device comprising the implantable medical material of any one of claims 1 to 5, and a holder for holding the implantable medical material.

7. An implantable medical device according to claim 6, wherein the holder is a stent.
